# EUROPEAN PATENT SPECIFICATION

(11) **EP 3 921 836 B1**
(45) Date of publication and mention of the grant of the patent: **11.03.2026**
(21) Application number: 20702333.4
(22) Date of filing: 05.02.2020
(51) Int. Cl.: G16C 20/20, B01D 15/34, G01N 21/552, G01N 30/00

(54) **A LABEL-FREE DETECTION METHOD FOR CHARACTERIZATION OF THE BEHAVIOR OF A COMPONENT IN A LIQUID**
ETIKETTENFREIES DETEKTIONSVERFAHREN ZUR CHARAKTERISIERUNG DES VERHALTENS EINER KOMPONENTE IN EINER FLÜSSIGKEIT
PROCÉDÉ DE DÉTECTION SANS ÉTIQUETTE POUR LA CARACTÉRISATION DU COMPORTEMENT D'UN COMPOSANT DANS UN LIQUIDE

(30) Priority: 05.02.2019 EP 19155496
(43) Date of publication of application: 15.12.2021
(73) Proprietor: Solve Research and Consultancy AB, 223 81 Lund (SE)
(72) Inventor: LEEMAN, Mats, 241 94 Hurva (SE); HANSSON, Sebastian, 224 74 Lund (SE); NILSSON, Lars, 225 93 Lund (SE)
(74) Representative: Høiberg P/S
(86) International application number: PCT/EP2020/052833
(87) International publication number: WO 2020/161173

(56) References cited:
- WO-A1-2011/093782
- DEN ENGELSMAN J. ET AL: "Strategies for the Assessment of Protein Aggregates in Pharmaceutical Biotech Product Development", PHARMACEUTICAL RESEARCH, vol. 28, no. 4, 23 October 2010 (2010-10-23), US, pages 920 - 933, XP055493713, ISSN: 0724-8741, DOI: 10.1007/s11095-010-0297-1
- SOME D.: "Light-scattering-based analysis of biomolecular interactions", BIOPHYSICAL REVIEWS, vol. 5, no. 2, 6 March 2013 (2013-03-06), DE, pages 147 - 158, XP055557891, ISSN: 1867-2450, DOI: 10.1007/s12551-013-0107-1
- RUNYON J. R. ET AL: "Preparation of Narrow Dispersity Gold Nanorods by Asymmetrical Flow Field-Flow Fractionation and Investigation of Surface Plasmon Resonance", ANALYTICAL CHEMISTRY, vol. 85, no. 2, 10 December 2012 (2012-12-10), US, pages 940 - 948, XP055654088, ISSN: 0003-2700, DOI: 10.1021/ac302571g
- FRAUNHOFER W. ET AL: "The use of asymmetrical flow field-flow fractionation in pharmaceutics and biopharmaceutics", EUROPEAN JOURNAL OF PHARMACEUTICS AND BIOPHARMACEUTICS, ELSEVIER SCIENCE PUBLISHERS B.V., AMSTERDAM, NL, vol. 58, no. 2, 1 September 2004 (2004-09-01), pages 369 - 383, XP004526319, ISSN: 0939-6411, DOI: 10.1016/J.EJPB.2004.03.034
- NISHI H. ET AL: "Label-Free Flow Cytometry Analysis of Subvisible Aggregates in Liquid IgG1 Antibody Formulations", JOURNAL OF PHARMACEUTICAL SCIENCES, vol. 103, no. 1, 11 November 2013 (2013-11-11), US, pages 90 - 99, XP055653762, ISSN: 0022-3549, DOI: 10.1002/jps.23782
- SUN K. ET AL: "Aptasensors for the selective detection of alpha-synuclein oligomer by colorimetry, surface plasmon resonance and electrochemical impedance spectroscopy", SENSORS AND ACTUATORS B: CHEMICAL, vol. 245, 1 June 2017 (2017-06-01), NL, pages 87 - 94, XP055594176, ISSN: 0925-4005, DOI: 10.1016/j.snb.2017.01.171
- XIAO Y. ET AL: "Label-Free Electronic Detection of Thrombin in Blood Serum by Using an Aptamer-Based Sensor", ANGEWANDTE CHEMIE INTERNATIONAL EDITION, vol. 44, no. 34, 22 August 2005 (2005-08-22), pages 5456 - 5459, XP009517783, DOI: 10.1002/anie.200500989

## Description

### Technical field

The present disclosure relates to a method and system for label-free characterization of the behavior of a component in a complex liquid sample, which does not require pre-treatment of either the complex liquid sample or the component.

### Background

Standard methods used to investigate the interactions of particles, such as biological therapeutics, are limited to either studying the drug in the formulation or the effects in the body - but not the drug behavior in its intended environment such as blood or other tissues. Protein-based drugs are a fast-growing sector within the pharmaceutical industry and are nowadays used in the treatment of numerous diseases. Therapeutic proteins are complex with respect to their large molecular size (cf. small-molecule drugs) and their secondary and tertiary structures that must be maintained to function effectively as drug molecules. These intrinsic properties of proteins are, together with environmental stress, part of the reasons that proteins are prone to aggregate in pharmaceutical processing, formulation, and during storage. The formation of protein aggregates can result in reduced drug efficacy and/or immunogenicity, which compromises patient safety (Frokjaer et al. 2005).

It is a significant challenge to predict the behavior of a therapeutic protein when in a complex matrix such as blood. Furthermore, it is challenging to predict the immunogenic potential of novel therapeutic proteins.

The primary method for determining size and amount of sub-visible aggregates is size-exclusion chromatography (SEC) in combination with suitable detectors. However, in recent years, SEC, although being well established, has been questioned as it can give erroneous estimates of the aggregate levels (Manning et al. 2014; Carpenter et al. 2010; USFDA, 2014). There is as such a significant need to understand how particles, such as the therapeutic proteins, behave in complex liquids, such as blood and other body fluids. The number of possible interactions between a component and further components of such a liquid, additionally makes computer simulations difficult, which otherwise have been a method which has had moderate success.

Regulatory authorities, such as the US Food and Drug Administration (FDA), now recommend conducting several complementary, orthogonal methods to verify the measurements. Analyses of protein aggregates in the finished formulation are important but even more central would be to understand the aggregation potential after administration, including interactions with blood components (Wang et al. 2012). Analyzing the aggregate formation of protein therapeutics after drug administration to the patient without tampering with the extracted sample has long been sought for and can only be achieved by analyzing blood either in vivo or ex vivo.

In the FDA industry guidance on aggregate analysis, no specific analytical method is recommended (Carpenter et al. 2010). Asymmetrical flow field-flow fractionation (AF4) has been utilized for the separation and characterization of blood plasma and lipoproteins (Madörinet al. 1997; Li et al. 1996; Park et al. 2002). However, in all cases, the plasma samples were prepared by some preparation methods (centrifugation and additives were added). This pre-treatment can, however, cause unintended artefacts such as unwanted loss of components, contamination, and protein aggregation.

Recently, Leeman et al. (2018) have found that it is possible to analyze the behavior of a fluorescently labelled antibody in serum, plasma, and whole blood by using asymmetrical flow field-flow fractionation (AF4) in combination with UV-vis, dRI, MALS and fluorescence detection. However, as stated by the authors, the fluorescence labelling introduces change in chemical properties of the antibody which, in turn, could influence its propensity to form aggregates.

Hence, a problem remains of being able to characterize the behavior of a certain component in a complex liquid, thereby mimicking the in vivo behavior of said component in said liquid, without labelling the component and without treating the complex liquid in a way that may affect the component's behavior.

### Summary of the invention

The invention is defined in the appended claims.

### Summary

The present inventors have surprisingly found that it is possible to characterize how a component, for example a drug or a nanoparticle, behaves in a complex fluid, for example blood, by combining a separation method with a detection method that is selective for the component under study. By the use of a detection method that is selective for the component under study without labelling said component more accurate characterizations can be realized. A further advantage by the use of a detection method that is selective for the component under study without labelling said component, is that pre-treatment of a complex liquid sample may be negated.

Preferably, field flow fractionation (FFF) is used followed by a detection method selective for the component under study, for example ELISA or SPR, without labelling the component and without pre-treating the complex liquid sample.

Furthermore, the method disclosed herein makes it possible to study and characterize how a certain drug will behave in the body of an individual, when it enters in contact with the individual's body fluid. With the disclosed method it is possible to determine if a certain drug has formed aggregates by dimerizing or multimerizing with copies of itself and/or other molecules once in contact with a body fluid, or if the drug is still in a monomeric form. The disclosed method also allows determining whether a certain drug will form aggregates by interacting with components of a body fluid once in contact with it. Furthermore, the disclosed method also allows determining whether a certain component will degrade, for example whether a drug part of an antibody-drug conjugate will be released once in contact with a body fluid.

It should be noted that the disclosed method may allow for characterization of a certain component without having to label said component. This is a substantial advantage over previously known methods because the presence of a label attached to the component that is to be characterized decreases the accuracy of said characterization.

One aspect of the present disclosure therefore relates to a label-free method for characterization of a first component in a complex liquid sample, the method comprising:
(a) providing a complex liquid sample comprising the first component;
(b) applying a field flow fractionation (FFF) to the sample;
(c) obtaining a plurality of fractions of the sample of (b);
(d) applying to the fractions a label-free detection method selective for the first component;
(e) obtaining an output signal from the label-free detection method;
(f) analyzing the output signal to determine presence of the first component within the fractions,

thereby characterizing the first component as being in a monomeric form and/or in an aggregate form,
wherein said monomeric form comprises or consists of the first component or a fragment of the first component, and
wherein said aggregate form comprises or consists of two or more copies of the first component, or the first component together with one or more further components of the complex liquid sample.

Another aspect of the present disclosure relates to a system for label-free characterization of a first component in a complex liquid, the system being capable of performing the method disclosed herein.

### Description of drawings

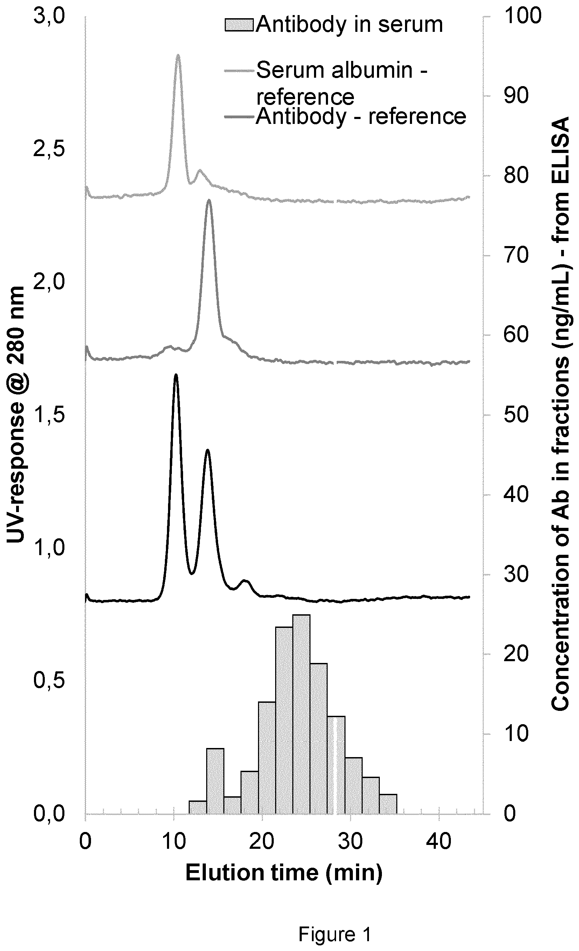

**Figure 1****:** Fractograms from the size separation of blood serum (black trace, left y-axis) showing the elution of the serum components using the non-selective UV-response at 280 nm. For reference the elution profile of a serum albumin and antibody standard is also shown. The bar graph (lowest in the figure) shows the concentration of the antibody of interest in the fractions eluted from the AF4 as detected with an ELISA method (selective for the antibody of interest).

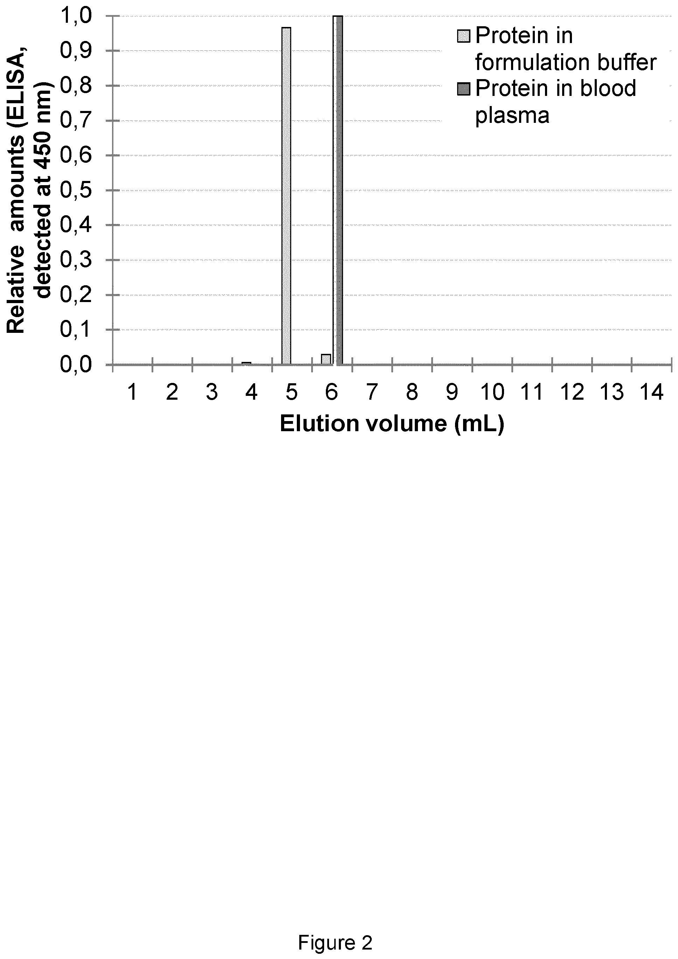

**Figure 2****:** Relative amounts of a protein in 14 different fractions obtained from the AF4 separation as either a) the protein in the formulation buffer (light grey bars) or b) the protein present plasma (dark grey bars). Detection was achieved with ELISA to allow selective detection in the complex plasma sample.

### Definitions

Complex liquid sample: as defined herein it refers to a liquid sample that comprises a plurality of components and that has a non-defined composition. The complex liquid sample may be a naturally occurring liquid, such as a body fluid, a food preparation, or other liquids.

Simple liquid sample: as defined herein it refers to an aqueous liquid sample that comprises only a few components, for example salts and/or surfactants. The simple liquid sample has a defined composition. Examples of simple liquid samples are given herein and may be saline solutions or buffer solutions, such as formulation buffers. The simple liquid sample is usually an aqueous liquid which is suitable for maintaining the first component of interest in its native, non-aggregated form.

First component: as defined herein it refers to a biological and/or chemical entity that may be naturally occurring or artificially made. The first component may itself consist of more monomers bound together. A non-exhaustive list of examples of biological and/or chemical entities that may exist in a multimeric form are antibodies, multimeric proteins, antibody-drug conjugates, viral particles. The first component may thus be a therapeutic molecule, a diagnostic molecule, an analyte, a particle, a nanoparticle.

Moiety: as used herein it refers to a chemical and/or biological entity such as a part or functional group of a molecule, a molecule, a particle, such as a nanoparticle or microparticle.

Label-free method: as defined herein it refers to a detection method that is capable of detecting a non-labelled first component in a liquid sample.

Selective method: as defined herein it refers to a detection method that is capable of detecting one or more specific analytes without interference from other components present in a sample comprising said analyte. Selective detection methods include, but are not limited to, enzyme-linked immunosorbent assay (ELISA), surface plasmon resonance (SPR), and mass spectrometry (MS). Examples of non-selective detection methods include, but are not limited to, ultraviolet (UV), refractive index (RI, and multiangle light scattering (MALS). Said selective method may as a consequence be described as a method selective for the one or more specific analytes.

Quantitative method: as defined herein it refers to a detection method that is capable of estimating, as a numerical value in appropriate units, the amount, or the concentration, of an analyte present in a sample.

Qualitative methods: as defined herein it refers to a detection method that is capable of providing information about the identity or form of one or more substances present. For instance, a chemist might be given an unknown solid sample and use qualitative analytical methods (for example NMR or IR spectroscopy) to identify compounds present, and thereafter quantitative techniques to determine the amount of each compound.

### Detailed description

The present disclosure relates to a label-free method for characterization of a first component in a complex liquid sample, the method comprising:
(a) providing a complex liquid sample comprising the first component;
(b) applying a field flow fractionation (FFF) to the sample;
(c) obtaining a plurality of fractions of the sample of (b);
(d) applying to the fractions a label-free detection method selective for the first component;
(e) obtaining an output signal from the label-free detection method;
(f) analyzing the output signal to determine presence of the first component within the fractions,

thereby characterizing the first component as being in a monomeric form and/or in an aggregate form,
wherein said monomeric form comprises or consists of the first component or a fragment of the first component, and
wherein said aggregate form comprises or consists of two or more copies of the first component, or the first component together with one or more further components of the complex liquid sample.

"A monomeric form" as used herein refers to a single unity or entity of the first component. Thus, if the first component is a multimeric protein, the monomeric form of said multimeric protein is the multimeric protein itself.

One of the problems solved by the present invention is that of characterizing whether a non-labelled target component that has been added to a certain complex liquid sample, usually a body fluid, interacts or does not interact with itself and/or with other components present in the complex liquid sample.

As explained in the "Background" section herein, methods exists in the art which are capable of analyzing whether a labelled-component interacts or does not interact with copies of itself and/or with other components present in a sample, but these methods are dependent on the target component being labelled, for example the target component may be attached to a fluorophore or radioisotope. This labelling may affect the behavior of the target component and therefore it may also affect whether the target component will interact or not with copies of itself and/or with other components present in the liquid, thereby resulting in false and/or unreliable results.

The present invention overcomes this problem by the providing a label-free method as disclosed herein.

One embodiment of the present disclosure involves providing a complex liquid sample comprising a first component. The complex liquid sample may stem from an artificial liquid or it may stem from a normally occurring liquid, such as a biological liquid, such as a mammalian body fluid. The characterization of the first component is carried out without the addition of labels to the sample. This ensures that the results of the characterization are not affected by any label that may have been attached to the sample.

### The first component

The present disclosure relates to a method for characterization of a first component in a complex liquid sample. In particular, the aim of the method is to characterize the behavior of said first component in a complex liquid sample.

The first component as defined herein may be a biological or chemical entity that is added to the complex liquid sample. It may also be said that the complex liquid sample is spiked with the first component.

The first component as defined herein may be a biological or chemical entity that is already present in the complex liquid at the time of obtaining a sample. For example, the first component may be a biological and/or chemical entity that was administered to a subject prior to obtaining a sample of a complex liquid of said subject.

Hence, in one embodiment the first component is added to the complex liquid prior to obtaining a complex liquid sample.

There is a need in characterizing how a certain component behaves when added to a complex liquid sample, for example for characterizing how a drug will behave when administered to an individual.

Hence, the first component may be any biological and/or chemical entity that may be used as medicament, in the formulation of a medicament, dietary supplement, food component, food supplement, as biomarker, as diagnostic biomarker and/or in the form of nanoparticles.

In one embodiment of the present disclosure, the first component interacts with copies of itself thereby forming dimers, trimers, multimers, or aggregates.

In one embodiment of the present disclosure, the first component and the one or more further components of the complex liquid sample may be identical to and/or different from each other.

In one embodiment of the present disclosure, the one or more further components of the complex liquid sample may be identical and/or different from each other.

In one embodiment of the present disclosure, the first component may interact with a number of components of the complex liquid sample which are identical and/or different to the first component, and further, the first component may interact with two or more further components wherein the two or more further components of the complex liquid sample may be identical and/or different from each other.

The first component may interact with several components. For example, the first component may interact directly with each and every one of the one or more further components. Alternatively, the first component may interact with one or more further components via another further component. Thus, an aggregate may be formed wherein the first component does not interact directly with all the components of the aggregate, but only with one or few of the components that form the aggregate.

In one embodiment of the present disclosure, the one or more further components are components that are usually found in the complex liquid sample.

In an embodiment of the present disclosure the first component is selected from the group consisting of a peptide, an antibody, a protein, an aggregation of proteins, an aggregation of antibodies, an antibody fragment, a coated nanoparticle, a non-coated nanoparticle, a virus, a virus-like particle, an exosome, a liposome, a vesicle, a biomarker, a ubiquitinated protein, a vaccine, an antigen, a microparticle, a polymeric microparticle and fragments thereof.

In one embodiment, the biomarker may be, but are not limited to, amyloid-beta, tau, alpha-synuclein or polyglutamine tract (PolyQ tract).

As known to the person skilled in the art, amyloid beta (Aβ or Abeta) denotes peptides of 36-43 amino acids that are crucially involved in Alzheimer's disease as the main component of the amyloid plaques which may be found in the brains of Alzheimer patients. The peptides derive from the amyloid precursor protein (APP), which is cleaved by beta secretase and gamma secretase to yield Aβ. Aβ molecules may aggregate to form flexible soluble oligomers which may exist in several forms. It is now believed that certain misfolded oligomers (known as "seeds") can induce other Aβ molecules to also take the misfolded oligomeric form, leading to a chain reaction akin to a prion infection. The oligomers are toxic to nerve cells. The other protein implicated in Alzheimer's disease, tau protein, also forms such prion-like misfolded oligomers, and there is some evidence that misfolded Aβ may induce tau to misfold.

As known to the person skilled in the art, tau protein is a highly soluble microtubule-associated protein tau (MAPT). In humans, these proteins are found mostly in neurons compared to non-neuronal cells. One of tau's main functions is to modulate the stability of axonal microtubules. Tau proteins interact with tubulin to stabilize microtubules and promote tubulin assembly into microtubules. Tau may control microtubule stability by isoforms and phosphorylation and defective tau proteins are related to diseases such as Alzheimer's disease and Parkinson's disease.

As known to the person skilled in the art, Alpha-synuclein is a protein that, in humans, is encoded by the SNCA gene. It is abundant in the brain while smaller amounts are found in the heart, muscles, and other tissues. In the brain, alpha-synuclein is found mainly at the tips of nerve cells (neurons) in specialized structures called presynaptic terminals. Within these structures, alpha-synuclein interacts with phospholipids and proteins Presynaptic terminals release chemical messengers, called neurotransmitters, from compartments known as synaptic vesicles. The release of neurotransmitters relays signals between neurons and is critical for normal brain function.

As known to the person skilled in the art, a polyglutamine tract or polyQ tract is a portion of a protein consisting of a sequence of several glutamine units. A tract typically consists of about 10 to a few hundred such units. Several
inheritable neurodegenerative disorders, the polyglutamine diseases, occur if a mutation causes a polyglutamine tract in a specific gene to become too long. Important examples of polyglutamine diseases are spinocerebellar ataxia and Huntington's disease.

In an embodiment of the present disclosure the first component has a biocorona, that is, the first component may be, at least partially, covered in a biocorona.

Components, such as nanoparticles introduced into a complex liquid may become coated with biomolecules present in the complex liquid, which result in formation of a nanoparticle-biocorona complex. The formation of a biocorona complex may alter a component's, such as a nanoparticle's, properties including, but not limited to, physicochemical characteristics, functionality, biodistribution and toxicity. The biocorona may comprise a first layer of biomolecules, usually referred to as hard biocorona, which interacts with the component's surface. Further, the biocorona may comprise a secondary layer, a soft biocorona, which may include macromolecules interacting with the hard biocorona.

In one embodiment, presence of a biocorona on the first component affects the output signal, such as the elution time and/or the signal amplitude obtained from the label-free detection method relative to the first component.

In one embodiment, presence of a biocorona and modification to a biocorona result in discrepancies in the output signal of the label-free detector. These discrepancies may be detected and analyzed by the method of the present disclosure in order to characterize the first component, such as the biocorona of the first component.

Changes to the signal include, but are not limited to, changes in temporal, oscillation, spatial and/or the amplitude of the signal, such as a different elution time. These changes may be determined by the method of the present disclosure and are part of the characterization of the first component.

The biocorona may further act to affect the interaction between the first component with copies of itself and with the one or more further components of the complex liquid sample.

In one embodiment of the present disclosure, the size of the first component is compatible with FFF.

Usually, FFF may be able to discriminate between analytes within certain size ranges, depending on the type of FFF.

In one embodiment, the first component comprises or consists of a particle having a diameter in a range between 1 nm and 10 µm, such as in a range between 1 nm and 1 µm, such as in a range between 1 nm and 800 nm, such as in a range between 1 nm and 600 nm, such as in a range between 1 nm and 400 nm, such as in a range between 1 nm and 200 nm, such as in a range between 1 nm and 100 nm, such as in a range between 1 nm and 80 nm, such as in a range between 1 nm and 50 nm, such as in a range between 1 nm and 10 nm, such as in a range between 10 nm and 10 µm, such as in a range between 50 nm and 10 µm, such as in a range between 100 nm and 10 µm, such as in a range between 200 nm and 10 µm, such as in a range between 400 nm and 10 µm, such as in a range between 600 nm and 10 µm, such as in a range between 800 nm and 10 µm, such as in a range between 1 µm and 10 µm.

In one embodiment, the first component has a molecular weight in a range between 1 kDa and 2 MDa, such as in a range between 1 kDa and 1,5 MDa, such as in a range between 1 kDa and 1 MDa, such as in a range between 1 kDa and 900 kDa, such as in a range between 1 kDa and 750 kDa, such as in a range between 1 kDa and 500 kDa, such as in a range between 1 kDa and 250 kDa, such as in a range between 1 kDa and 100 kDa, such as in a range between 1 kDa and 80 kDa, such as in a range between 1 kDa and 60 kDa, such as in a range between 1 kDa and 50 kDa, such as in a range between 1 kDa and 40 kDa, such as in a range between 1 kDa and 20 kDa, such as in a range between 1 kDa and 15 kDa, such as in a range between 1 kDa and 10 kDa.

### Complex liquid and further components

One of the advantages of the method of the present disclosure is that it allows characterization of the first component in a complex liquid without labelling said first component. This means that the disclosed method makes it possible to simulate how the first component, for example a molecule that is being developed to be a therapeutic, would behave in a real situation, for example in the blood stream of an individual.

In one embodiment of the present disclosure, the complex liquid sample is a mammalian body fluid.

In one embodiment of the present disclosure, the complex liquid sample is an organoid composition.

In one embodiment of the present disclosure, the mammalian body fluid is obtained from human, rat, mouse, dog, primate, rabbit or horse.

In one embodiment, the complex liquid is a human body fluid.

In one embodiment, the complex liquid is a body fluid obtained from apes.

In one embodiment of the present disclosure, the mammalian body fluid is selected from the group consisting of blood, serum, plasma, cerebrospinal fluid (CSF), urine, gastric acid, sputum, saliva, bile, lymph, peritoneal fluid, pleural fluid (usually referred to as pleural effusion), amniotic fluid or a peripheral blood mononuclear cell (PBMC) composition.

Blood plasma is a protein-rich solution in which white and red blood cells, as well as platelets, are suspended, and serum is the remaining fluid after removal of the clot from whole blood with principally the same composition as plasma with the exception that the fibrinogens and clotting factors are absent. The protein concentration in plasma/serum is approximately 60-80 mg/mL of which about 50-60% are albumins and 40% globulins (10-20% immunoglobulin G, IgG). The size distribution of blood components ranges from small molecules and ions (< 1 nm) to about 15 µm for white blood cells. Due to the complex nature and the large size range of components in blood, such samples are difficult to analyze and extensive sample pre-treatment is generally included. Typical sample preparation steps involve centrifugation, extraction, and filtration (Pitt al. 2016).

In one embodiment of the present disclosure the complex liquid sample is not pretreated prior to applying the field flow fractionation (FFF).

In one embodiment of the present disclosure the complex liquid sample is not pretreated by centrifugation, extraction, and/or filtration prior to applying the field flow fractionation (FFF).

In one embodiment of the present disclosure the complex liquid sample is diluted prior to applying the field flow fractionation (FFF). Said dilution may comprise addition of a buffer, such as a formulation buffer to said complex liquid sample. An example of a formulation buffer is an isotonic aqueous solution of phosphate and sodium chloride salts, such as phosphate buffered saline.

The dilution may have the purpose of adjusting the viscosity of the sample and so improving the separation of the components present in the complex liquid fluid by FFF. In one embodiment of the present disclosure, any of the one or more further components is each independently a biological moiety and/or a chemical moiety.

In one embodiment of the present disclosure, any of the one or more further components is a moiety commonly found in said complex liquid, such as commonly found in said body fluid.

In one embodiment of the present disclosure, any of the one or more further components is a moiety that has been added to said complex liquid. This can be important to study, for example, how different drugs interact with each other once they have been administered to an individual and are in contact with a certain complex fluid, for example blood, saliva, CSF and/or any other of the complex liquids mentioned herein.

In one embodiment of the present disclosure, any of the one or more further components is each independently a cell, a peptide, a polypeptide, an enzyme, a lipid molecule, a carbohydrate molecule, a vesicle, a surfactant, a drug, a nanoparticle, an adjuvant, a plastic microparticle and fragments thereof and combinations thereof.

In one embodiment of the present disclosure, the one or more further components comprise ions. For example, with the method disclosed herein it is possible to characterize a protein as interacting with calcium ions or other ions that binds to said protein thereby causing a change in 2D and/or 3D conformation.

### Field Flow Fractionation (FFF)

In one embodiment, a step of the method for characterization of a first component in a complex liquid sample disclosed herein, comprises applying field flow fractionation (FFF) to said sample so that a plurality of fractions of said sample are obtained.

In one embodiment of the present disclosure, the shear stress exerted on the first component, when the field flow fractionation is applied, is not sufficient for shear degradation.

FFF is an established separation technique. Components within the complex liquid sample may be separated according to properties of the components: the components may be separated in space or time, and/or the components may have different elution times.

Asymmetrical flow field-flow fractionation (AF4) is a method in which separation is achieved by applying an external field (cross flow) in a ribbon-like open channel without a stationary phase. Due to the absence of a stationary phase with which the components of a sample interact, several problems related
to SEC are alleviated including minimization of nonspecific protein adsorption, structural deformation at the surface and high shear forces which may result in degradation, aggregation or other changes of analytes.

The properties which affect the separation of the components depend on the specific type of FFF method which is used for the separation. One property which may affect the separation of the components is, in one embodiment of the present disclosure, size.

Hence, larger components may be separated relative to smaller components.

Several FFF methods are known to the person of skills in the art.

In one embodiment of the present disclosure, the FFF is selected from the group consisting of: asymmetric flow field-flow fractionation (AF4), sedimentation field-flow fractionation (SdFFF), hollow fiber flow field-flow fractionation (HF5), thermal field-flow fractionation (ThFFF), centrifugal field-flow fractionation (CFFF), split flow thin-cell fractionation (SPLITT), dielectrophoretic field-flow fractionation (DEP-FFF), acoustic field-flow fractionation (AcFFF), gravitational field-flow fractionation (GFFF), electrical asymmetric flow field-flow fractionation (EAF4), electric field-flow fractionation (EFFF), magnetic field-flow fractionation (MFFF), steric field-flow fractionation (StFFF), flow field-flow fractionation (FFFF), chemical field-flow fractionation (CFFF), high temperature asymmetric flow field-flow fractionation (HTAF4), medium temperature asymmetric flow field-flow fractionation (MTAF4) and micro asymmetric flow field-flow fractionation (mAF4).

In one embodiment of the present disclosure, the FFF is AF4.

In one embodiment, the method of the present disclosure comprises obtaining a plurality of fractions of the complex liquid sample comprising the first component, wherein said fractions are obtained following field flow fractionation.

In some embodiments, all fractions obtained following field flow fractionation, that is the equivalent of the entire sample, are further analyzed with the label-free detection method. This is for example done when it is not known when the first component will be eluted, and hence it is not known in which fractions the first component will be.

In some embodiments, only those fractions comprising the first component are further analyzed with the label-free detection method. This is for example possible if the elution time of the first component, as well as the elution times of the possible aggregated, bound and/or degraded forms, are already known, such as if it is known which fractions will comprise the first component, for example based on previous results obtained with the same method.

### Label-free detection method

The label-free method for characterization of a first component in a complex liquid sample comprises the steps of (e) applying a label-free detection method selective for the first component to the fractions; and (f) obtaining an output signal from the label-free detection method.

In a preferred embodiment of the present disclosure, the detection method is a selective detection method. Preferably, said detection method is capable of detecting the first component without interference from other components, such as other particles, present in the complex liquid sample.

In one embodiment of the present disclosure, the detection method is a quantitative detection method. Preferably, said detection method is capable of estimating, as a numerical value in appropriate units, the amount, or the concentration, of a first component present in the complex liquid sample.

The fraction(s) of the sample obtained after field flow fractionation are further analyzed with a label-free detection method being selective for the first component. The label-free detection method provides an output signal, which is then analyzed thereby characterizing the first component as:
- being in a monomeric form, and/or
- in an aggregate form,
wherein said monomeric form comprises or consists of the first component or a fragment of the first component, and wherein said aggregate form comprises or consists of two or more copies of the first component, or the first component together with one or more further components of the complex liquid sample.

The label-free detection method provides an output signal, which is analyzed thereby also characterizing the first component as:
- non interacting;
- interacting with copies of itself; and/or
- interacting with the one or more further components of the complex liquid sample.

In some embodiments of the present disclosure the first component does not interact with either itself or the one or more further components of the complex liquid sample, hence it is eluted in one single fraction.

In some embodiments of the present disclosure, part of the first component may interact with copies of itself to form a dimer or a larger multimer, part of the first component may interact with one component of the complex liquid sample, for example with a cell found in blood, part of the first component may interact with a further component present in the complex liquid sample, for example with an enzyme, and part of the first component is left unreacted. In such a situation, the first component will be eluted in different fractions obtained after field flow fractionation of the sample, and the different forms in which the first component is found will be detected by the label-free detection method selective for said first component.

In one embodiment of the present disclosure, the output signal obtained for the first component is dependent on the number and/or the nature of interaction of the first component with copies of itself and/or with the one or more further components.

In one embodiment of the present disclosure, the output signal obtained for the first component is dependent on the form in which the first component exists in the complex liquid sample.

In one embodiment of the present disclosure, the output signal obtained from the label-free detection method for the first component that interacts with the one or more further components is different from the output signal obtained for the first component that does not interact with the one or more further components.

In one embodiment of the present disclosure, the output signal comprises elution time and/or signal amplitude.

The output signal may have been acquired at different positions of one or multiple detectors such that the signal has a spatial difference in the acquisition. Moreover, the output signal may have a temporal discrepancy or change in the shape of the signal, such as a change in the signal amplitude, depending on whether the first component interacts or does not interact with itself (copies of the first component) and/or with the one or more further components.

The differences in elution time and/or signal amplitude may be related to how properties, such as size, charge, density, shape, conformation of the first component are modified by the interaction with copies of itself and/or the one or more further components.

The output signal may comprise information related to the first component and interaction of the first component, such as if the first component interacts with the one or more further components or not, such as if the first component is in an aggregate form, in a monomeric form (or as a fragment). In addition to a certain elution time and signal amplitude, the output signal may further have a wave shape, such as a sine wave.

Additionally, the output signal can be an analogue signal or a digital signal having either a continuous or a discrete time and it may be quantified, such as amplitude quantification.

The output signal may be an electric signal, but may alternatively be based on other physical quantities, including but not limited to, energy, force, torque, light, motion and position.

The output signal may be generated by an active label-free method wherein an excitation signal from an external power source is required for the operations. Further, the output signal may be generated by a passive label-free method wherein a signal, such as an electric current, may be generated in response to an external stimulus without the need for an additional energy.

In one embodiment of the present disclosure, the label-free detection method involves the use of a biological and/or chemical moiety which selectively targets the first component, an aggregate comprising the first component and/or a fragment of the first component.

In one embodiment of the present disclosure, the biological and/or chemical moiety may be a biologically occurring ligand of the first component. Said biologically occurring ligand may in some embodiments be the biological counterpart of the first component that is being characterized. For example, where the first component is an antibody, an antigen known to bind to said antibody may be attached on a surface of a detector of the label-free detection method.

In one embodiment of the present disclosure, the biological and/or chemical moiety is an antibody, an antigen, a selective polymer, a molecular imprinted polymer (MIP) or a gold surface, such as that of a gold nanoparticle.

In one embodiment of the present disclosure, the biological and/or chemical moiety is immobilized on a surface of a detector of any one of the label-free detection methods and/or on a surface of a free-floating particle, wherein said free-floating particle is added to one of the fraction obtained in step (d) prior to applying the label-free detection method.

In one embodiment of the present disclosure, the free-floating particle comprises a magnetic particle, a radioisotope, a fluorophore, an enzyme, a metal particle, a quantum dot and/or a fluorescent protein.

In one embodiment of the present disclosure, the label-free detection method selective for the first component is one, or a combination of, surface plasmon resonance (SPR), enzyme-linked immunosorbent assay (ELISA), mass spectrometry (MS), quartz crystal microbalance (QCM), quartz crystal microbalance with dissipation monitoring (QCM-D), electrochemiluminescence (ECL), bio-layer interferometry (BLI), electric potential integrated circuit (EPIC), immuno-polymerase chain reaction (immuno-PCR), immunoassays such as Singulex Erenna^{®} and Gyrolab^{®}, meso scale discovery (MSD), radioimmunoassay (RIA), AlphaLISA^{®}, AlphaScreen^{®}, AlphaPlex^{®}, dissociation-enhanced lanthanide fluorescence immunoassay (DELFIA) or impedance measurements such as xCELLigence^{®}.

In one embodiment of the present disclosure, the label-free detection method selective for the first component is ELISA.

In one embodiment of the present disclosure, the label-free detection method selective for the first component is SPR.

In one embodiment of the present disclosure, the label-free detection method selective for the first component is mass spectrometry (MS),

In one embodiment of the present disclosure, the label-free detection method selective for the first component is ECL.

The label-free method for characterization of a first component in a complex liquid sample may further comprise a second detection step, wherein said detection step may comprise a non-selective detection method.

In one embodiment, the method of the present disclosure further comprises a second detection step, said second detection step consisting of one, or a combination of, multi-angle light scattering (MALS) or differential refractive index (dRI) or refractive index (RI) or ultraviolet-visible spectroscopy (UV-VIS) or optical absorption spectrophotometer (OAS) or multi-angle laser light scattering (MALLS) or graphite furnace atomic absorption spectrometer (GF-AAS) or ultra violet light (UV) detector, a fluorescent detector, low-angle laser light scattering (LALLS), optical microscopy such as super resolution microscopy, electron microscopy such as scanning electron microscopy (SEM) or transmission electron microscopy (TEM), mass spectrometry, such as electrospray ionization mass spectrometry (ESI-MS), inductively coupled plasma mass spectrometry (ICP-MS) or matrix-assisted laser desorption/ionization mass spectrometry (MALDI-MS), flow cytometry, coulter counter, impedance measurements, nanoparticle tracking analysis (NTA), dynamic light scattering (DLS), laser diffraction, atomic force microscopy (AFM), x-ray diffraction, static light scattering, nephelometry, capillary electrophoresis, gel electrophoresis, differential mobility analysis, small-angle x-ray scattering (SAXS), cross-flow filtration, ultracentrifugation, centrifugal sedimentation, rate-zonal centrifugation, nuclear magnetic resonance spectroscopy, neutron activation analysis, x-ray fluorescence, x-ray spectroscopy, x-ray crystallography, energy dissipative x-ray analysis (EDXA), electron energy loss spectroscopy (EELS), thermogravimetric analysis (TGA), atomic absorption spectroscopy (AAS), atomic emission spectroscopy (AES), two-dimensional gel electrophoresis (2DE), fluorescence energy transfer (FRET).

In one embodiment of the present disclosure, the second detection step is conducted before step (e) applying a label-free detection method selective for the first component to the fractions. This may allow a first selection of the fractions that most likely comprise the first component both as it is in a monomeric form, and where it interacts with copies of itself and/or with the one or more further components of the complex liquid sample, that is in an aggregate form or as a fragment.

In one embodiment of the present disclosure, the second detection step is conducted after step (e) applying a label-free detection method selective for the first component to the fractions. This may allow further characterization of those fractions identified as comprising the first component as it is in its monomeric form; and where it interacts with copies of itself and/or with the one or more further components on the complex liquid sample, that is in an aggregate form and/or as a fragment.

In one embodiment of the present disclosure, the first component is characterized as being in an aggregate form, and the method further comprises analyzing the one or more further components of the complex liquid sample that are comprised in the aggregate.

In one embodiment of the present disclosure, the first component is characterized as being in an aggregate form, and the method further comprises identifying the one or more further components of the complex liquid sample that are comprised in the aggregate. The person skilled in art knows which analytical chemistry techniques are available for analysis and identification of biological and/or chemical moieties and would use the most suitable ones depending on the nature of the component to be analyzed.

### Characterization of the first component

The characterization of the first component in a complex liquid sample may comprise characterizing whether said first component
- Does not interact with copies of itself and/or with one or more further components of the complex liquid sample,
- Interacts with copies of itself, and/or
- Interacts with one or more further components of the complex liquid sample.

The aim of the method disclosed herein is to characterize a first component in a complex liquid sample and in particular:
- characterizing the first component as being in a monomeric form, wherein said monomeric form comprises or consists of the first component or a fragment of the first component and/or
- characterizing the first component as being in an aggregate form, wherein said aggregate form comprises or consists of two or more copies of the first component, or the first component together with one or more further components of the complex liquid sample.

In one embodiment of the present disclosure, the characterization of the first component in a complex liquid sample is a determination of a multimerization state of the first component. The multimerization state of the first component may be the first component being in a monomeric form. The multimerization state of the first component may also be the first component being in a dimeric or multimeric form, wherein said first component interacts with one or more copies of the first component thereby forming a multimer.

In one embodiment of the present disclosure, the monomeric form comprises or consists of the first component.

In one embodiment, the first component is characterized as being in a monomeric form, wherein said monomer is only a fraction of the first component. Hence, in ne embodiment of the present disclosure, the first component is degraded to two or more fragments as a result of the interaction of the first component with one or more further components of the complex liquid.

In one embodiment of the present disclosure, the monomeric form comprises or consists of a fragment of the first component, wherein said fragment is the result of an interaction between the first component and one or more further components of the complex liquid sample.

In one embodiment of the present disclosure, the characterization of the first component in a complex liquid sample is a determination of an aggregation state of the first component. The aggregation state of the first component may be the first component being in a non-aggregated state, such as when the first component does not interact with other components in the complex liquid. The aggregation state of the first component may be the first component being in an aggregated state, such as when the first component interacts with one or more further components in the complex liquid.

In one embodiment of the present disclosure, the aggregate form comprises or consists of the first component together with one or more further components, and said aggregate form is the result of an interaction between the first component and one or more further components of the complex liquid sample.

In one embodiment of the present disclosure, the aggregate form comprises or consists of the first component together with one or more further components, wherein said further components are identical to and/or different from each other.

In one embodiment of the present disclosure, the aggregate form comprises reversible interactions.

In one embodiment of the present disclosure, the aggregate form comprises irreversible interactions.

This characterization comprises analyzing the output signal obtained from the label-free detection method relative to all those fraction of the sample that comprises the first component in an monomeric form, in an aggregate form, in a multimeric form and/or as a fragment.

In one embodiment of the present disclosure, the method further comprises comparing the output signal, for example the elution time and/or the signal amplitude, obtained for the first component with one or more control values.

The one or more control values may be provided reference values of the first component, as known from previous experiments and/or from scientific literature.

The one or more control values may be the average elution time values and/or the average signal amplitude values relative to the first component, wherein said control values are obtained by characterizing the first component using the method disclosed herein, with the difference that for obtaining the one or more control values the first component is added to a simple liquid sample, instead of to a complex liquid sample. This ensures that the behavior of the first component in the control sample is not affected by possible interactions with other components found in the simple liquid sample, as the simple liquid sample is in fact a buffer, such as a formulation buffer, as defined herein.

In one embodiment of the present disclosure, the simple liquid sample is a liquid comprising or consisting of water and/or a formulation buffer.

In one embodiment of the present disclosure, the simple liquid sample is an artificial solution.

In one embodiment of the present disclosure, the simple liquid sample is a liquid comprising or consisting of PBS, water, saline catholyte, salts, one or more carbohydrates, one or more polymers, one or more surfactants and/or a glycerol solution.

In one embodiment of the present disclosure, the simple liquid sample is a liquid, wherein the first component is substantially in its native and/or non-aggregated state.

In one embodiment of the present disclosure, the first component has been artificially added to the simple liquid sample.

In one embodiment of the present disclosure, the interaction between the first component and the one or more further components is aggregation, degradation and/or binding. For example, interaction of the first component with copies of itself and/or with the one or more further components may result in the formation of an aggregate.

In one embodiment of the present disclosure, the aggregation is dimerization, trimerization, tetramerization or multimerization.

In one embodiment of the present disclosure, the interaction is a chemical and/or physical interaction.

In one embodiment of the present disclosure, the interaction comprises or consists of covalent bonds, ionic bonds, hydrogen bonds, hydrophobic interactions, electrostatic interactions, van der Waals forces, metallic bonds and/or dipole-dipole interactions.

In one embodiment of the present disclosure, each interaction between the first component and another copy of said first component, or with one or more further components of the complex liquid may be a reversible interaction.

In one embodiment of the present disclosure, each interaction between the first component and another copy of said first component, or with one or more further components of the complex liquid may be an irreversible interaction.

One example of a first component that may be degraded when added to the complex sample is a protein or peptide that is cleaved by a protease present in the complex sample.

A first component may behave differently in different complex liquids, it may form a large number of interaction in a type of complex liquid, and few and/or different interactions in another type of complex liquid. This can be exemplified by an antibody-drug conjugate (ADC) that is meant to release the drug in the brain: ideally, said ADC is expected to stay in an aggregated form in blood, but to degrade and thereby release the drug once it reaches the cerebrospinal fluid. The method of the present disclosure is capable of analyzing and determining the behavior of an ADC in different complex liquids, such as in blood, plasma, serum, cerebrospinal fluid, and thereby predicting how the ADC will behave, for example if the drug will reach the target tissue in the right form, once administered to the individual.

### System

One aspect of the present disclosure relates to a system for label-free characterization of a first component in a complex liquid sample, the system being capable of performing the method disclosed herein.

In one embodiment of the present disclosure, the system comprises or consists of a field-flow fractionation (FFF) separator and a device for label-free detection of the first component, wherein said FFF separator is coupled to said label-free detection device, and wherein said label-free detection is selective for the first component.

In one embodiment of the present disclosure, the FFF separator is coupled to said label-free detection device off-line, at-line, on-line or in-line.

By off-line coupling it is intended that the FFF separator and the label-free detection device are physically distinguished units and the sample has to be actively transferred from one device to the second. The FFF separator and the label-free detection device may for example be located in different buildings.

By at-line coupling it is intended that the FFF separator and the label-free detection device are physically distinguished units and the sample has to be actively transferred, for example manually, or with the help of a robot, from one device to the second. The FFF separator and the label-free detection device may for example be located adjacent to each other.

By on-line coupling it is intended that the FFF separator and the label-free detection device are physically distinguished units arranged so that the sample is automatically transferred, for example via tubing, from one device to the second. The FFF separator and the label-free detection device may for example be located adjacent to each other.

By in-line coupling it is intended that the FFF separator and the label-free detection device are physically connected and the sample is automatically transferred from one device to the second.

In one embodiment of the present disclosure the system comprises a processor configured for analyzing an output signal, such as an output signal of a detector, to determine presence of the first component, such as within a fraction.

In one embodiment of the present disclosure the system comprises a flow generator, for inducing fluid flow in the system, such as flow of a sample in a separation device.

In one embodiment of the present disclosure the system comprises means for displaying to a user, a result of an analysis of an output signal.

### Examples

### Example 1. Antibody in Plasma: AF4/ELISA: Binding study heterocomplex.

A binding study was performed in which a blood serum sample containing an antibody and the antibody bound to a serum component (i.e. a heterocomplex with the antibody) was size separated on AF4. The AF4 size was able to separate the serum components, such as serum albumin from the non-heterocomplexed antibody (i.e. free antibody) as well as from the complexed antibody. The fractions were collected for analysis by enzyme-linked immunosorbent assay (ELISA).

### Experimental details:

A therapeutically interesting antibody (10 µL of a 1 mg/mL solution) was spiked into blood plasma (90 µL) from different mammalian species, including human. The plasma samples were mixed by gentle swirling and incubated for 30 minutes at 37 °C in a capped plastic vial. Following the incubation period, the spiked plasma samples were diluted with an isotonic aqueous solution of phosphate and sodium chloride salts and size separated on asymmetrical flow field-flow fractionation (AF4). The AF4 separation and analysis was performed on an Eclipse II (Wyatt technology) in connection with a 1100-series LC-system consisting of an ERC-3415 vacuum degasser (ERC), a G1311A pump, G1329A auto sampler, and a G1315A diode array (UV) detector (Agilent technologies). A Dawn Heleos II multi-angle light scattering (MALS) and Optilab t-Rex differential refractive index (dRI) detector were connected on-line (Wyatt technology) after the channel. The UV detector monitored the wavelength at 280 nm. The MALS utilized a laser with 658 nm wavelength and measure scattered light with 17 detectors in the aqueous mobile phase. The dRI detector utilizes a lamp monitoring at 658 nm wavelength. Fractionation was run at ambient temperature (approximately 22 °C). The separation method used a detector flow rate of 0.50 mL/min giving a system pressure of approximately 3 bar. The asymmetrical flow field-flow fractionation channel was a Wyatt SC channel fitted with a low molecular weight cut-off membrane. Fractions of the antibody and antibody-heterocomplex were collected and analyzed off-line by enzyme-linked immunosorbent assay (ELISA) appropriate and selective for the target antibody.

Results: The data presented in Figure 1 show that the antibody binds to large components of the blood plasma forming hetero-complexes (aggregates).

Conclusion: A method combining AF4 and ELISA was used to characterize and study an antibody in blood plasma and to determine that it interacts with particles (moieties) of the blood plasma thereby forming aggregates with various component of the blood plasma. The AF4 was able to characterize and study the antibody's interaction with particles in plasma and ELISA was able to selectively detect the antibody in the plasma fractions.

### Example 2. Protein in Plasma: AF4/ELISA: Study of protein aggregation in plasma

The aim of this study was to use AF4 in combination with ELISA to characterize and study protein aggregation in plasma, and to determine the specificity of the protein of interest in plasma.

### Experimental details:

The protein (10 µL of a stock solution) was spiked into blood plasma (150 µL), mixed by gentle swirling and incubated for 30 minutes at 37 °C in capped plastic vials. Following the incubation the spiked plasma sample was diluted with an isotonic aqueous solution of phosphate and sodium chloride salts and size separated on asymmetrical flow field-flow fractionation (AF4). The AF4 separation and analysis was performed on an Eclipse II (Wyatt technology) in connection with a 1100-series LC-system consisting of an ERC-3415 vacuum degasser (ERC), a G1311A pump, G1329A auto sampler, and a G1315A diode array (UV) detector (Agilent technologies). The UV detector monitored the wavelength at 280 nm. Fractionation was run at ambient temperature (approximately 22 °C). The separation method used a detector flow rate of 0.5 mL/min giving a system pressure of approximately 3 bar. The asymmetrical flow field-flow fractionation channel was a Wyatt SC channel fitted with a low molecular weight cut-off membrane. Fractions of the protein was collected and analyzed off-line by enzyme-linked immunosorbent assay (ELISA). The ELISA was performed on the fractions either un-diluted or after dilution up to 27 times (depending on fraction). The protein concentration was obtained from a calibration curve covering the 0 - 3 ppb range.

Results: The plasma components including the protein of interest were separated according to size by AF4 and fractions collected. The fractions were analyzed by ELISA allowing selective detection of the protein, thus enabling determination of the size distribution of the protein in plasma. It was found that the protein was eluting in fraction 6.

For comparison the protein was separated by AF4 when present in formulation buffer (same buffer used in Example 1 above). Fractions were collected and analyzed. In the formulation buffer the protein was known to be present as a monomeric species. The protein monomeric form (in formulation buffer) was found to elute in fraction 5. Hence, the protein elutes in a later fraction indicating that it has an apparent larger size when in a blood plasma matrix. This is likely due to protein interactions either with the protein itself or some of the blood plasma components.

Conclusion: A method combining AF4 and ELISA was used to characterize and study a protein in plasma and to determine that it forms aggregates in plasma whereas it is found in monomeric form in formulation buffer.

### Example 3. Serum Albumin in Serum: AF4/SPR: Proof of concept study

A characterization study of the protein serum albumin in blood serum samples was conducted as follows.

### Experimental details:

Solution of human serum albumin as well as human blood serum was diluted with an isotonic aqueous solution of phosphate and sodium chloride salts and size separated on asymmetrical flow field-flow fractionation (AF4). The AF4 separation and analysis was performed on an Eclipse II (Wyatt technology) in connection with a 1100-series LC-system consisting of an ERC-3415 vacuum degasser (ERC), a G1311A pump, G1329A auto sampler, and a G1315A diode array (UV) detector (Agilent technologies). The UV detector monitored the wavelength at 280 nm. Fractionation was run at ambient temperature (approximately 22 °C). The asymmetrical flow field-flow fractionation channel was a Wyatt SC channel fitted with a low molecular weight cut-off membrane. The AF4-UV setup was connected to a surface plasmon resonance (SPR) (Bionavis). Fractions from the AF4 was loaded onto a loop (on-line) and measured upon by the SPR-device. The SPR was fitted with a serum albumin selective chip allowing the selective detection of human serum albumin.

Conclusion: A method combining AF4 and SPR was used to characterize and study serum albumin in serum and to determine the specificity of serum albumin in serum. The blood serum components were separated according to size by AF4 and fractions were switched onto an SPR instrument for selective detection of serum albumin. It was shown that serum albumin could be detected in the serum fractions from the AF4.

### Example 4. Antibody in Serum: AF4/SPR (Trastuzumab)

An antibody (Trastuzumab, obtained from Apoteket) was stressed by subjecting it to elevated temperature causing the formation of sub-micron aggregates. The stressed antibody was spiked into human blood serum and mixed by gentle swirling and incubated for 30 minutes at ambient temperature in a capped plastic vial. Following the incubation the spiked serum samples was diluted with an isotonic aqueous solution of phosphate and sodium chloride salts and size separated on asymmetrical flow field-flow fractionation (AF4). The AF4 separation and analysis was performed on an Eclipse II (Wyatt technology) in connection with a 1100-series LC-system consisting of an ERC-3415 vacuum degasser (ERC), a G1311A pump, G1329A auto sampler, and a G1315A diode array (UV) detector (Agilent technologies). A Dawn Heleos II multi-angle light scattering (MALS) and Optilab t-Rex differential refractive index (dRI) detector were connected on-line (Wyatt technology) after the channel. The UV detector monitored the wavelength at 280 nm. Fractionation was run at ambient temperature (approximately 22 °C). The separation method used a detector flow rate of 0.50 mL/min. The asymmetrical flow field-flow fractionation channel was a Wyatt SC channel fitted with a low molecular weight cut-off membrane. Fractions of the antibody and antibody aggregate was collected and analyzed off-line by surface plasmon resonance (SPR) appropriate and selective for the target antibody.

Conclusion: A combined AF4-SPR analysis was conducted. AF4 allowed characterizing and studying the antibody and antibody aggregates in serum and the following SPR allowed to determine the specificity of the antibody and to verify the presence of aggregates in serum.

### References

Frokjaer S, Otzen DE. Protein drug stability: a formulation challenge. Nat Rev Drug Disc. 2005;4(4):298-306.
Manning RR, Holcomb RE,Wilson GA, Henry CS, Manning MC. Review of orthogonal methods to SEC for quantitation and characterization of protein aggregates. Biopharm Int. 2014;27(12):32.
Carpenter JF, Randolph TW, Jiskoot W, Crommelin DJA, Middaugh CR, Winter G. Potential inaccurate quantitation and sizing of protein aggregates by size exclusion chromatography: essential need to use orthogonal methods to assure the quality of therapeutic protein products. J Pharm Sci. 2010;99(5):2200-8.
USFDA (2014) Guidance for industry immunogenicity assessment for therapeutic protein products.
Wang W, Singh SK, Li N, Toler MR, King KR, Nema S. Immunogenicity of protein aggregates-concerns and realities. Int J Pharm. 2012;431 (1-2): 1-11.
Madörin M, Van Hoogevest P, Hilfiker R, Langwost B, Kresbach GM, EhratM, et al. Analysis of drug/plasma protein interactions by means of asymmetrical flow field-flow fractionation. Pharm Res. 1997;14(12):1706-12.
Li P, Giddings JC. Isolation and measurement of colloids in human plasma by membrane-selective flow field-flow fractionation: lipoproteins and pharmaceutical colloids. J PharmSci. 1996;85(8):895-8.
Park I, Paeng KJ, Yoon Y, Song JH, Moon MH. Separation and selective detection of lipoprotein particles of patients with coronary artery disease by frit-inlet asymmetrical flow field-flow fractionation. J Chromatogr B Anal Technol Biomed Life Sci. 2002;780(2):415-22.
Leeman M, Choi J., Hansson S., Ulmius Storm M. and Nilsson L. Proteins and antibodies in serum, plasma, and whole blood-size characterization using asymmetrical flow field-flow fractionation (AF4). Anal Bioanal Chem (2018) 410:4867-4873.
Pitt WG, AlizadehM, Husseini GA, McClellan DS, BuchananCM, Bledsoe CG, et al. Rapid separation of bacteria from blood-review and outlook. Biotechnol Prog. 2016;32(4):823-39.

## Claims

1. A label-free method for characterization of a first component in a complex liquid sample, the method comprising:
(a) providing a complex liquid sample comprising the first component, said complex liquid sample being a mammalian body fluid selected from the group consisting of blood, serum, plasma, cerebrospinal fluid (CSF), urine, gastric acid, sputum, saliva, bile, lymph, peritoneal fluid, pleural fluid, amniotic fluid and a peripheral blood mononuclear cell (PBMC) composition;
(b) applying a field flow fractionation (FFF) to the sample;
(c) obtaining a plurality of fractions of the sample of (b);
(d) applying to the fractions a label-free detection method selective for the first component;
(e) obtaining an output signal from the label-free detection method;
(f) analyzing the output signal to determine presence of the first component within the fractions,
thereby characterizing the first component as being in a monomeric form and/or in an aggregate form,
wherein said monomeric form comprises or consists of the first component or a fragment of the first component, and
wherein said aggregate form comprises or consists of two or more copies of the first component, or the first component together with one or more further components of the complex liquid sample.

2. The method of claim 1, wherein the detection method is a quantitative detection method such as wherein the detection method is capable of estimating, as a numerical value in appropriate units, the amount, or the concentration, of a first component present in the complex liquid sample.

3. The method according to any of the preceding claims, wherein the detection method is capable of detecting the first component without interference from other components, such as other particles, present in the complex liquid sample.

4. The method according to any one of the preceding claims, wherein the characterization is a determination of a multimerization state of the first component and/or a determination of an aggregation state of the first component.

5. The method according to any one of the preceding claims, wherein the monomeric form comprises or consists of a fragment of the first component, wherein said fragment is the result of an interaction between the first component and one or more further components of the complex liquid sample.

6. The method according to any one of the preceding claims, wherein the aggregate form comprises or consists of two or more copies of the first component, and/or the first component together with one or more further components, and wherein said aggregate form is the result of an interaction between the two or more copies of the first component and/or between the first component and one or more further components of the complex liquid sample.

7. The method according to any of claims 5-6, wherein the interaction between the first component and the one or more further components is a chemical and/or physical interaction.

8. The method according to any one of the preceding claims, wherein the output signal obtained for the first component is dependent on the form in which the first component exists in the complex liquid sample.

9. The method according to any one of the preceding claims, wherein the output signal comprises elution time and/or signal amplitude of the first component.

10. The method according to any one of the preceding claims, wherein the first component is selected from the group consisting of a peptide, an antibody, a protein, an aggregation of proteins, an aggregation of antibodies, an antibody fragment, a coated nanoparticle, a non-coated nanoparticle, a virus, a virus-like particle, an exosome, a vesicle, a liposome, a biomarker, a ubiquitinated protein, a vaccine, an antigen, a microparticle, a polymeric microparticle and fragments thereof, wherein the biomarker is amyloid-beta, tau, alpha-synuclein or polyglutamine (PolyQ), and/or.
wherein any of the one or more further components is each independently a biological moiety and/or a chemical moiety, such as wherein any of the one or more further components is each independently a cell, a peptide, a polypeptide, a lipid molecule, a carbohydrate molecule, a vesicle, a surfactant, a drug, a nanoparticle, an adjuvant, a plastic microparticle and fragments thereof and combinations thereof.

11. The method according to any one of the preceding claims, further comprising comparing the elution time and/or the signal amplitude obtained for the first component with one or more control values, wherein the one or more control values are provided reference values of the first component, or wherein the one or more control values are the average elution time values and/or the average signal amplitude values relative to the first component, wherein said control values are obtained by characterizing the first component in a simple liquid sample.

12. The method according to any one of the preceding claims, wherein shear stress exerted on the first component during step (b) is not sufficient for shear degradation.

13. The method according to any one of the preceding claims, wherein the FFF is selected from the group consisting of: asymmetric flow field-flow fractionation (AF4), sedimentation field-flow fractionation (SdFFF), hollow fiber flow field-flow fractionation (HF5), thermal field-flow fractionation (ThFFF), centrifugal field-flow fractionation (CFFF), split flow thin-cell fractionation (SPLITT), dielectrophoretic field-flow fractionation (DEP-FFF), acoustic field-flow fractionation (AcFFF), gravitational field-flow fractionation (GFFF), electrical asymmetric flow field-flow fractionation (EAF4), electric field-flow fractionation (EFFF), magnetic field-flow fractionation (MFFF), steric field-flow fractionation (StFFF), flow field-flow fractionation (FFFF), chemical field-flow fractionation (CFFF), high temperature asymmetric flow field-flow fractionation (HTAF4), medium temperature asymmetric flow field-flow fractionation (MTAF4) and micro asymmetric flow field-flow fractionation (mAF4) and/or wherein the label-free detection method is one, or a combination of, surface plasmon resonance (SPR), enzyme-linked immunosorbent assay (ELISA), mass spectrometry (MS), quartz crystal microbalance (QCM), quartz crystal microbalance with dissipation monitoring (QCM-D), electrochemiluminescence (ECL), bio-layer interferometry (BLI), electric potential integrated circuit (EPIC), immuno-polymerase chain reaction (immuno-PCR), immunoassays, meso scale discovery (MSD), radioimmunoassay (RIA), dissociation-enhanced lanthanide fluorescence immunoassay (DELFIA) or impedance measurements.

14. The method according to any of the preceding claims, wherein the label-free detection method involves the use of a biological and/or chemical moiety which selectively targets the first component, an aggregate comprising the first component and/or a fragment of the first component, wherein the biological and/or chemical moiety is immobilized on a surface of a detector of any one of the label-free detection methods and/or on a surface of a free-floating particle, wherein said free-floating particle is provided to the fraction obtained in step (c).

15. A system for label-free characterization of a first component in a complex liquid, the system being capable of performing the method defined in any one of claims 1-14, wherein said system comprises or consists of a field-flow fractionation (FFF) separator and a device for the label-free detection of the first component, wherein said FFF separator is coupled to said label-free detection device off-line, at-line, on-line or in-line, and wherein said label-free detection is selective for the first component.

## Patentansprüche

1. Ettikettenfreies Verfahren zur Charakterisierung einer ersten Komponente in einer komplexen Flüssigkeitsprobe, wobei das Verfahren Folgendes umfasst:
(a) Bereitstellen einer komplexen Flüssigkeitsprobe, die die erste Komponente umfasst, wobei die komplexe Flüssigkeitsprobe eine Säugetierkörperflüssigkeit ist, ausgewählt aus der Gruppe bestehend aus Blut, Serum, Plasma, Zerebrospinalflüssigkeit (CSF), Urin, Magensäure, Sputum, Speichel, Galle, Lymphe, Peritonealflüssigkeit, Pleuraflüssigkeit, Amnionflüssigkeit und einer Zusammensetzung aus peripheren mononukleären Blutzellen (PBMC);
(b) Anwenden einer Feldflussfraktionierung (FFF) auf die Probe;
(c) Erhalten einer Vielzahl von Fraktionen der Probe aus (b);
(d) Anwenden eines ettikettenfreien Detektionsverfahrens, das für die erste Komponente selektiv ist, auf die Fraktionen;
(e) Erhalten eines Ausgangssignals aus dem ettikettenfreien Detektionsverfahren;
(f) Analysieren des Ausgangssignals, um das Vorhandensein der ersten Komponente innerhalb der Fraktionen zu bestimmen,
wodurch die erste Komponente als in einer monomeren Form und/oder in einer aggregierten Form vorliegend charakterisiert wird,
wobei die monomere Form die erste Komponente oder ein Fragment der ersten Komponente umfasst oder daraus besteht, und
wobei die aggregierte Form zwei oder mehr Kopien der ersten Komponente oder die erste Komponente zusammen mit einer oder mehreren weiteren Komponenten der komplexen Flüssigkeitsprobe umfasst oder daraus besteht.

2. Verfahren nach Anspruch 1, wobei das Detektionsverfahren ein quantitatives Detektionsverfahren ist, bei dem beispielsweise das Detektionsverfahren in der Lage ist, die Menge oder die Konzentration einer ersten Komponente, die in der komplexen Flüssigkeitsprobe vorhanden ist, als numerischen Wert in geeigneten Einheiten zu schätzen.

3. Verfahren nach einem der vorhergehenden Ansprüche, wobei das Detektionsverfahren in der Lage ist, die erste Komponente ohne Störung durch andere Komponenten, wie beispielsweise andere Partikel, die in der komplexen Flüssigkeitsprobe vorhanden sind, zu detektieren.

4. Verfahren nach einem der vorhergehenden Ansprüche, wobei die Charakterisierung eine Bestimmung eines Multimerisierungszustands der ersten Komponente und/oder eine Bestimmung eines Aggregatzustands der ersten Komponente ist.

5. Verfahren nach einem der vorhergehenden Ansprüche, wobei die monomere Form ein Fragment der ersten Komponente umfasst oder daraus besteht, wobei das Fragment das Ergebnis einer Wechselwirkung zwischen der ersten Komponente und einer oder mehreren weiteren Komponenten der komplexen Flüssigkeitsprobe ist.

6. Verfahren nach einem der vorhergehenden Ansprüche, wobei die aggregierte Form zwei oder mehr Kopien der ersten Komponente und/oder die erste Komponente zusammen mit einer oder mehreren weiteren Komponenten umfasst oder daraus besteht, und wobei die aggregierte Form das Ergebnis einer Wechselwirkung zwischen den zwei oder mehr Kopien der ersten Komponente und/oder zwischen der ersten Komponente und einer oder mehreren weiteren Komponenten der komplexen Flüssigkeitsprobe ist.

7. Verfahren nach einem der Ansprüche 5-6, wobei die Wechselwirkung zwischen der ersten Komponente und der einen oder den mehreren weiteren Komponenten eine chemische und/oder physikalische Wechselwirkung ist.

8. Verfahren nach einem der vorhergehenden Ansprüche, wobei das für die erste Komponente erhaltene Ausgangssignal von der Form abhängt, in der die erste Komponente in der komplexen Flüssigkeitsprobe vorliegt.

9. Verfahren nach einem der vorhergehenden Ansprüche, wobei das Ausgangssignal die Elutionszeit und/oder die Signalamplitude der ersten Komponente umfasst.

10. Verfahren nach einem der vorhergehenden Ansprüche, wobei die erste Komponente aus der Gruppe ausgewählt ist, bestehend aus einem Peptid, einem Antikörper, einem Protein, einer Aggregation von Proteinen, einer Aggregation von Antikörpern, einem Antikörperfragment, einem beschichteten Nanopartikel, einem unbeschichteten Nanopartikel, einem Virus, einem virusähnlichen Partikel, einem Exosom, einem Vesikel, einem Liposom, einem Biomarker, einem ubiquitinierten Protein, einem Impfstoff, einem Antigen, einem Mikropartikel, einem polymeren Mikropartikel und Fragmenten davon, wobei der Biomarker Amyloid-beta, Tau, Alpha-Synuclein oder Polyglutamin (PolyQ) ist, und/oder
wobei jede der einen oder mehreren weiteren Komponenten unabhängig voneinander eine biologische Einheit und/oder eine chemische Einheit ist, wobei jede der einen oder der mehreren weiteren Komponenten unabhängig voneinander eine Zelle, ein Peptid, ein Polypeptid, ein Lipidmolekül, ein Kohlenhydratmolekül, ein Vesikel, ein Tensid, ein Arzneimittel, ein Nanopartikel, ein Adjuvans, ein Kunststoffmikropartikel und Fragmente davon und Kombinationen davon ist.

11. Verfahren nach einem der vorhergehenden Ansprüche, ferner umfassend das Vergleichen der für die erste Komponente erhaltenen Elutionszeit und/oder Signalamplitude mit einem oder mehreren Kontrollwerten, wobei der eine oder die mehreren Kontrollwerte bereitgestellte Referenzwerte der ersten Komponente sind oder wobei der eine oder die mehreren Kontrollwerte die durchschnittlichen Elutionszeitwerte und/oder die durchschnittlichen Signalamplitudenwerte relativ zur ersten Komponente sind, wobei die Kontrollwerte durch Charakterisieren der ersten Komponente in einer einfachen Flüssigkeitsprobe erhalten werden.

12. Verfahren nach einem der vorhergehenden Ansprüche, wobei die während Schritt (b) auf die erste Komponente ausgeübte Scherspannung nicht für eine Scherdegradation ausreicht.

13. Verfahren nach einem der vorhergehenden Ansprüche, wobei die FFF aus der Gruppe ausgewählt ist, bestehend aus: asymmetrischer Fluss-Feld-Fluss-Fraktionierung (AF4), Sedimentations-Feld-Fluss-Fraktionierung (SdFFF), Hohlfaserfluss-Feld-Fluss-Fraktionierung (HF5), thermischer Feld-Fluss-Fraktionierung (ThFFF), zentrifugaler Feld-Fluss-Fraktionierung (CFFF), Split-Flow-Thin-Cell-Fraktionierung (SPLITT), dielektrophoretische Feld-Fluss-Fraktionierung (DEP-FFF), akustische Feld-Fluss-Fraktionierung (AcFFF), Gravitations--Feld-Fluss-Fraktionierung (GFFF), elektrische asymmetrische Feld-Fluss-Fraktionierung (EAF4), elektrische Feld-Fluss-Fraktionierung (EFFF), Magnetfeld-Feld-Fluss-Fraktionierung (MFFF), sterische Feld-Fluss-Fraktionierung (StFFF), Fluss-Feld-Fluss-Fraktionierung (FFFF), chemische Feld-Fluss-Fraktionierung (CFFF), asymmetrische Hochtemperatur-Feld-Fluss-Fraktionierung (HTAF4), asymmetrische Mitteltemperatur-Feld-Fluss-Fraktionierung (MTAF4) und asymmetrische Mikro-Feld-Fluss-Fraktionierung (mAF4) und/oder wobei das ettikettenfreie Detektionsverfahren eines von oder eine Kombination aus Oberflächenplasmonenresonanz (SPR), Enzymimmunoassay (ELISA), Massenspektrometrie (MS), Quarzkristallmikrowaage (QCM), Quarzkristallmikrowaage mit Dissipationsüberwachung (QCM-D), Elektrochemilumineszenz (ECL), Bio-Layer-Interferometrie (BLI), elektrischer Potentialintegrierter Schaltkreis (EPIC), Immun-Polymerase-Kettenreaktion (Immun-PCR), Immunoassays, Meso Scale Discovery (MSD), Radioimmunoassay (RIA), dissoziationsverstärkter Lanthanid-Fluoreszenz-Immunoassay (DELFIA) oder Impedanzmessungen.

14. Verfahren nach einem der vorhergehenden Ansprüche, wobei das ettikettenfreie Detektionsverfahren die Verwendung einer biologischen und/oder chemischen Einheit umfasst, die selektiv auf die erste Komponente, ein Aggregat, das die erste Komponente und/oder ein Fragment der ersten Komponente umfasst, abzielt, wobei die biologische und/oder chemische Einheit auf einer Oberfläche eines Detektors eines der ettikettenfreien Detektionsverfahren und/oder auf einer Oberfläche eines frei schwebenden Partikels immobilisiert ist, wobei das frei schwebende Partikel der in Schritt (c) erhaltenen Fraktion zugeführt wird.

15. System zur ettikettenfreien Charakterisierung einer ersten Komponente in einer komplexen Flüssigkeit, wobei das System in der Lage ist, das in einem der Ansprüche 1 bis 14 definierte Verfahren durchzuführen, wobei das System einen Feldflussfraktionierungsseparator(FFF)-Separator und eine Vorrichtung zur ettikettenfreien Detektion der ersten Komponente umfasst oder daraus besteht, wobei der FFF-Separator offline, at-line, online oder inline gekoppelt ist und wobei die ettikettenfreie Detektion selektiv für die erste Komponente ist.

## Revendications

1. Procédé sans étiquette pour la caractérisation d'un premier composant dans un échantillon liquide complexe, le procédé comprenant :
(a) la fourniture d'un échantillon liquide complexe comprenant le premier composant, ledit échantillon liquide complexe étant un fluide corporel de mammifère choisi parmi le groupe constitué de sang, de sérum, de plasma, de liquide céphalo-rachidien (LCR), d'urine, d'acide gastrique, d'expectorations, de salive, de bile, de lymphe, de liquide péritonéal, de liquide pleural, de liquide amniotique et d'une composition de cellules mononucléées du sang périphérique (PBMC) ;
(b) l'application d'un fractionnement par flux de champ (FFF) à l'échantillon ;
(c) l'obtention d'une pluralité de fractions de l'échantillon de (b) ;
(d) l'application aux fractions d'un procédé de détection sans étiquette sélectif pour le premier composant ;
(e) l'obtention d'un signal de sortie à partir du procédé de détection sans étiquette ;
(f) l'analyse du signal de sortie pour déterminer la présence du premier composant dans les fractions,
caractérisant ainsi le premier composant comme étant sous forme monomère et/ou sous forme agrégée,
dans lequel ladite forme monomère comprend ou est constituée du premier composant ou d'un fragment du premier composant, et dans lequel ladite forme agrégée comprend ou est constituée de deux ou plusieurs copies du premier composant, ou du premier composant ainsi que d'un ou plusieurs autres composants de l'échantillon liquide complexe.

2. Procédé selon la revendication 1, dans lequel le procédé de détection est un procédé de détection quantitative tel que dans lequel le procédé de détection est capable d'estimer, sous forme de valeur numérique dans des unités appropriées, la quantité ou la concentration d'un premier composant présent dans l'échantillon liquide complexe.

3. Procédé selon l'une quelconque des revendications précédentes, dans lequel le procédé de détection est capable de détecter le premier composant sans interférence provenant d'autres composants, tels que d'autres particules, présents dans l'échantillon liquide complexe.

4. Procédé selon l'une quelconque des revendications précédentes, dans lequel la caractérisation est une détermination d'un état de multimérisation du premier composant et/ou une détermination d'un état d'agrégation du premier composant.

5. Procédé selon l'une quelconque des revendications précédentes, dans lequel la forme monomère comprend ou est constituée d'un fragment du premier composant, dans lequel ledit fragment est le résultat d'une interaction entre le premier composant et un ou plusieurs autres composants de l'échantillon liquide complexe.

6. Procédé selon l'une quelconque des revendications précédentes, dans lequel la forme agrégée comprend ou est constituée de deux copies ou plus du premier composant, et/ou du premier composant ainsi que d'un ou plusieurs autres composants, et dans lequel ladite forme agrégée est le résultat d'une interaction entre les deux copies ou plus du premier composant et/ou entre le premier composant et un ou plusieurs autres composants de l'échantillon liquide complexe.

7. Procédé selon l'une quelconque des revendications 5 et 6, dans lequel l'interaction entre le premier composant et les un ou plusieurs autres composants est une interaction chimique et/ou physique.

8. Procédé selon l'une quelconque des revendications précédentes, dans lequel le signal de sortie obtenu pour le premier composant dépend de la forme sous laquelle le premier composant existe dans l'échantillon liquide complexe.

9. Procédé selon l'une quelconque des revendications précédentes, dans lequel le signal de sortie comprend le temps d'élution et/ou l'amplitude de signal du premier composant.

10. Procédé selon l'une quelconque des revendications précédentes, dans lequel le premier composant est choisi parmi le groupe constitué d'un peptide, d'un anticorps, d'une protéine, d'un agrégat de protéines, d'un agrégat d'anticorps, d'un fragment d'anticorps, d'une nanoparticule enrobée, d'une nanoparticule non enrobée, d'un virus, d'une particule de type viral, d'un exosome, d'une vésicule, d'un liposome, d'un biomarqueur, d'une protéine ubiquitinée, d'un vaccin, d'un antigène, d'une microparticule, d'une microparticule polymérique et de fragments de ceux-ci, dans lequel le biomarqueur est l'amyloïde-bêta, la protéine tau, l'alpha-synucléine ou la polyglutamine (PolyQ), et/ou dans lequel l'un quelconque des un ou plusieurs autres composants est indépendamment une fraction biologique et/ou une fraction chimique, telle que dans lequel chacun des un ou plusieurs autres composants est indépendamment une cellule, un peptide, un polypeptide, une molécule lipidique, une molécule glucidique, une vésicule, un tensioactif, un médicament, une nanoparticule, un adjuvant, une microparticule de plastique et des fragments de ceux-ci et des combinaisons de ceux-ci.

11. Procédé selon l'une quelconque des revendications précédentes, comprenant également la comparaison du temps d'élution et/ou de l'amplitude de signal obtenus pour le premier composant avec une ou plusieurs valeurs de commande, dans lequel les une ou plusieurs valeurs de commande sont des valeurs de référence fournies du premier composant, ou dans lequel les une ou plusieurs valeurs de commande sont les valeurs moyennes du temps d'élution et/ou les valeurs moyennes de l'amplitude de signal par rapport au premier composant, dans lequel lesdites valeurs de commande sont obtenues en caractérisant le premier composant dans un échantillon liquide simple.

12. Procédé selon l'une quelconque des revendications précédentes, dans lequel la contrainte de cisaillement exercée sur le premier composant pendant l'étape (b) n'est pas suffisante pour une dégradation par cisaillement.

13. Procédé selon l'une quelconque des revendications précédentes, dans lequel le FFF est choisi parmi le groupe constitué de : fractionnement par flux de champ asymétrique (AF4), fractionnement par flux de champ par sédimentation (SdFFF), fractionnement par flux de champ à fibres creuses (HF5), fractionnement par flux de champ thermique (ThFFF), fractionnement par flux de champ centrifuge (CFFF), fractionnement par flux divisé à cellules minces (SPLITT), fractionnement par flux de champ diélectrophorétique (DEP-FFF), fractionnement par flux de champ acoustique (AcFFF), fractionnement par flux de champ gravitationnel (GFFF), fractionnement par flux de champ asymétrique électrique (EAF4), fractionnement par flux de champ électrique (EFFF), fractionnement par flux de champ magnétique (MFFF), fractionnement par flux de champ stérique (StFFF), fractionnement par flux de champ (FFFF), fractionnement par flux de champ chimique (CFFF), fractionnement par flux de champ asymétrique à haute température (HTAF4), fractionnement par flux de champ asymétrique à température moyenne (MTAF4) et micro-fractionnement par flux de champ asymétrique (mAF4) et/ou dans lequel le procédé de détection sans étiquette est l'un, ou une combinaison, d'une résonance plasmonique de surface (SPR), d'un dosage immuno-enzymatique (ELISA), d'une spectrométrie de masse (MS), d'une microbalance à cristal de quartz (QCM), d'une microbalance à cristal de quartz avec surveillance de la dissipation (QCM-D), d'une électrochimiluminescence (ECL), d'une interférométrie de couche biologique (BLI), d'un circuit intégré de potentiel électrique (EPIC), d'une réaction en chaîne par polymérase immunologique (immuno-PCR), d'immunoessais, d'une découverte à l'échelle méso (MSD), d'un radioimmunoessai (RIA), d'un immunoessai de fluorescence de lanthanide à dissociation améliorée (DELFIA) ou de mesures d'impédance.

14. Procédé selon l'une quelconque des revendications précédentes, dans lequel le procédé de détection sans étiquette implique l'utilisation d'une fraction biologique et/ou chimique qui cible sélectivement le premier composant, un agrégat comprenant le premier composant et/ou un fragment du premier composant, dans lequel la fraction biologique et/ou chimique est immobilisée sur une surface d'un détecteur de l'un quelconque des procédés de détection sans étiquette et/ou sur une surface d'une particule en suspension, dans lequel ladite particule en suspension est fournie à la fraction obtenue à l'étape (c).

15. Système de caractérisation sans étiquette d'un premier composant dans un liquide complexe, le système étant capable de réaliser le procédé défini selon l'une quelconque des revendications 1 à 14, dans lequel ledit système comprend ou est constitué d'un séparateur à fractionnement par flux de champ (FFF) et d'un dispositif pour la détection sans étiquette du premier composant, dans lequel ledit séparateur FFF est couplé audit dispositif de détection sans étiquette hors ligne, sur place, en ligne ou in situ, et dans lequel ladite détection sans étiquette est sélective pour le premier composant.
